(19)

(11) **EP 3 120 802 B1**

(12) **EUROPÄISCHE PATENTSCHRIFT**

(45) Veröffentlichungstag und Bekanntmachung des Hinweises auf die Patenterteilung:
**23.05.2018 Patentblatt 2018/21**

(51) Int Cl.:
***A61C 8/00*** *(2006.01)* ***A61B 17/16*** *(2006.01)*
***A61C 1/00*** *(2006.01)* ***A61C 1/18*** *(2006.01)*

(21) Anmeldenummer: **15177428.8**

(22) Anmeldetag: **20.07.2015**

(54) **MEDIZINISCHE, INSBESONDERE DENTALE, VORRICHTUNG ZUR BESTIMMUNG DER QUALITÄT EINES KNOCHENS**

MEDICAL, IN PARTICULAR DENTAL DEVICE, FOR DETERMINING THE QUALITY OF A BONE

DISPOSITIF MÉDICAL, NOTAMMENT DENTAIRE, DESTINÉ À L'ÉVALUATION DE LA QUALITÉ D'UN OS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**25.01.2017 Patentblatt 2017/04**

(60) Teilanmeldung:
**18157376.7**

(73) Patentinhaber: **W & H Dentalwerk Bürmoos GmbH**
**5111 Bürmoos (AT)**

(72) Erfinder:
- **PLOY, Gernot**
  **5111 Bürmoos (AT)**
- **BRUGGER, Dr., Wilhelm**
  **5071 Wals-Siezenheim (AT)**

(74) Vertreter: **Benda, Ralf**
**W & H Dentalwerk Bürmoos GmbH**
**Ignaz-Glaser-Strasse 53**
**5111 Bürmoos (AT)**

(56) Entgegenhaltungen:
**WO-A1-00/15138** **WO-A1-2013/050851**
**WO-A2-2015/014771** **CN-A- 101 166 464**
**US-A1- 2011 245 833**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 120 802 B1

**Beschreibung**

Die Erfindung betrifft eine medizinische, insbesondere dentale oder dentalchirurgische, Vorrichtung zur Bestimmung der Qualität eines Knochens durch Schneiden eines Gewindes in den Knochen mit einem rotierenden Gewindeelement mit zumindest einer Schneide.

Eine derartige Vorrichtung ist aus der Patentanmeldung WO 2014/076653 A1 bekannt. Aus den Schriften US 2011/0245833 A1 und WO 2013/050851 A1 sind orthopädische Vorrichtungen bekannt, welche beim Bohren in einen Knochen die Eindringtiefe des Werkzeugs in den Knochen und das dabei aufgebrachte Drehmoment bzw. daraus abgeleitet die Knochendichte bestimmen und auf einer Anzeige darstellen.

Es ist Aufgabe der vorliegenden Erfindung, eine medizinische, insbesondere dentale oder dentalchirurgische, Vorrichtung zur Bestimmung der Qualität eines Knochens durch Schneiden eines Gewindes in den Knochen mit einem rotierenden Gewindeelement mit zumindest einer Schneide zu schaffen, die dem Anwender eine prägnante, schnell erfassbare und die wichtigsten Daten enthaltende Information über die ermittelte Knochenqualität, insbesondere auch über das qualitative Tiefenprofil des Knochens, bietet.

Diese Aufgabe wird durch eine medizinische, insbesondere dentale oder dentalchirurgische, Vorrichtung zur Bestimmung der Qualität eines Knochens gemäß Anspruch 1 gelöst. Vorteilhafte Weiterbildungen sind in den Unteransprüchen beschrieben.

Die medizinische, insbesondere dentale oder dentalchirurgische, Vorrichtung zur Bestimmung der Qualität eines Knochens durch Schneiden eines Gewindes in den Knochen mittels einem rotierenden Gewindeelement mit zumindest einer Schneide umfasst eine Steuervorrichtung, die eine erste elektrische Kontaktvorrichtung zur Verbindung mit einem motorischen Antrieb, eine zweite Kontaktvorrichtung zur Verbindung mit einer Anzeigeeinheit und einen Messschaltkreis, der operativ mit der ersten elektrischen Kontaktvorrichtung und der zweiten Kontaktvorrichtung verbunden ist, aufweist. Der Messschaltkreis ist ausgebildet:

(i) über die erste elektrische Kontaktvorrichtung Werte des Motorstroms zu bestimmen, mit dem der motorische Antrieb zum rotierenden Antreiben des mit dem motorischen Antrieb verbindbaren Gewindeelements versorgt wird, wobei die Motorstromwerte korrelierend oder im Wesentlichen proportional zu übertragenen Drehmomentwerten des motorischen Antriebs sind und wobei die Motorstromwerte oder Drehmomentwerte ein Maß für die Qualität eines Knochens sind,

(ii) die Eindringtiefe des Gewindeelements in den Knochen zu überwachen und / oder zu ermitteln und

(iii) Messsignale zu erzeugen, welche die bestimmten Motorstromwerte, die zu den Motorstromwerten korrelierenden oder im Wesentlichen proportionalen Drehmomentwerte oder davon abgeleitete Parameter mit der Eindringtiefe in Bezug setzen, und diese Messsignale über die zweite Kontaktvorrichtung an die Anzeigeeinheit zu übertragen, so dass die Anzeigeeinheit auf Basis der übertragenen Messsignale den Zusammenhang zwischen den bestimmten Motorstromwerten, den zu den Motorstromwerten korrelierenden oder im Wesentlichen proportionalen Drehmomentwerten und / oder den davon abgeleiteten Parametern, insbesondere der Qualität eines Knochens, und der Eindringtiefe wiedergibt.

Die Darstellung des Zusammenhangs zwischen den bestimmten Motorstromwerten, den zu den Motorstromwerten korrelierenden oder im Wesentlichen proportionalen Drehmomentwerten und / oder den davon abgeleiteten Parametern, insbesondere der Qualität eines Knochens, und der Eindringtiefe auf einer Anzeigeeinheit ermöglicht dem Anwender mit einem Blick die Qualität des Knochens zu erkennen, insbesondere den Verlauf oder Änderungen der Knochenqualität mit fortschreitender Eindringtiefe, und darauf basierend Entscheidungen für die nächsten Behandlungsschritte zu treffen.

Die von dem Messschaltkreis bestimmten Motorstromwerte sind ein Maß für die Qualität des Knochens, in den das Gewindeelement eingedreht wird. Die Qualität des Knochens, in den das Gewindeelement eingedreht wird, ist insbesondere zu den jeweiligen bestimmten Motorstromwerten korrelierend oder im Wesentlichen proportional und / oder ist von den jeweiligen bestimmten Motorstromwerten herleitbar, zum Beispiel über eine in der Steuervorrichtung vorgesehene oder dem Messschaltkreis zugeordnete Vergleichstabelle, in welcher unterschiedliche Motorstromwerte unterschiedlichen Knochenqualitäten zugeordnet sind. Die Steuervorrichtung oder der Messschaltkreis ist somit insbesondere dazu ausgebildet, die von dem Messschaltkreis bestimmten Motorstromwerten mit den in der Vergleichstabelle hinterlegten Motorstromwerten und / oder Knochenqualitäten zu vergleichen oder in Beziehung zu setzen.

Die erste elektrische Kontaktvorrichtung zur Verbindung mit einem motorischen Antrieb ist vorzugsweise über zumindest eine Versorgungs- oder Steuerleitung mit dem motorischen Antrieb verbunden. Vorzugsweise ist die erste elektrische Kontaktvorrichtung als lösbare Verbindung ausgebildet. Vorzugsweise ist an dem motorischen Antrieb ein Kupplungsanschluss zur Verbindung mit der ersten elektrischen Kontaktvorrichtung, insbesondere über die zumindest eine Versorgungs- oder Steuerleitung, vorgesehen. Vorzugsweise umfasst die erste elektrische Kontaktvorrichtung mehrere elektrische Kontakte, welche, insbesondere über die zumindest eine Versorgungs- oder Steuerleitung, mit elektrischen Kontakten des motorischen Antriebs verbunden sind.

Die zweite Kontaktvorrichtung zur Verbindung mit einer Anzeigeeinheit ist zum Beispiel als drahtgebundene, insbesondere lösbare, Kontaktvorrichtung ausgebildet. Vorzugsweise verbindet zumindest eine Versorgungs- oder Steuerleitung, über welche insbesondere die im Vorstehenden genannten Messsignale des Messschaltkreises an die Anzeigeeinheit übertragbar sind, die zweite Kontaktvorrichtung mit der Anzeigeeinheit oder mit einer an der Anzeigeeinheit vorgesehenen Kontaktvorrichtung. Die Messsignale umfassen insbesondere elektrische Signale, so dass die zweite Kontaktvorrichtung und die Kontaktvorrichtung der Anzeigeeinheit besonders bevorzugt als elektrische Kontaktvorrichtungen ausgebildet sind.

Alternativ ist die zweite Kontaktvorrichtung zur Verbindung mit einer Anzeigeeinheit als drahtlose Kontaktvorrichtung ausgebildet, insbesondere zum Übertragen elektromagnetischer Wellen, zum Beispiel von Radiowellen. Vorzugsweise umfasst die zweite Kontaktvorrichtung somit zumindest eine Sendeeinheit und die Anzeigeeinheit zumindest eine Empfangseinheit, so dass die im Vorstehenden genannten Messsignale des Messschaltkreises oder die darauf basierenden elektromagnetischen Wellen an die Anzeigeeinheit übertragbar sind. Die zweite Kontaktvorrichtung umfasst insbesondere auch eine Wandlereinheit zum Wandeln der elektrischen Messsignale des Messschaltkreises in elektromagnetischer Wellen, insbesondere Radiowellen.

Der Messschaltkreis ist vorzugsweise durch einen Mikroprozessor und eine entsprechende Software realisiert. Es ist jedoch auch denkbar, den Messschaltkreis durch Hardware, insbesondere Logikbausteine, oder durch eine Kombination von einem Mikroprozessor mit Software und Hardware auszubilden. Besonders bevorzugt ist der Mikroprozessor oder Mikrocontroller Teil der Steuervorrichtung und umfasst insbesondere weitere Steuer- oder Regelschaltungen, zum Beispiel zum Steuern oder Regeln des motorischen Antriebs und / oder der Anzeigeeinheit und / oder zum Verarbeiten von über ein oder mehrere Bedienelemente oder Stellelemente, insbesondere durch den Anwender, erzeugbare Signale. Die Bedienelemente oder Stellelemente sind insbesondere Teil der medizinischen, insbesondere dentalen, Vorrichtung und zum Beispiel an der Steuervorrichtung und / oder an der Anzeigeeinheit vorgesehen und / oder damit verbunden.

Die Ermittlung der Eindringtiefe (des fortschreitenden Eindringens) des Gewindeelements in den Knochen kann durch verschiedene Vorrichtungen und Verfahren erfolgen, zum Beispiel durch eine optische Messung / optische Messvorichtung oder durch eine Impedanzmessung / Impedanzmessvorrichtung oder mittels Schall / einer Schallmessvorrichtung. Alle diese alternativen Ausführungen benötigen jedoch zusätzliche Bauteile, zum Beispiel von Strahlungs-, Schall- oder Stromquellen, so dass eine Implementierung in eine Vorrichtung zur Bestimmung der Knochenqualität technisch kompliziert und teuer ist. Die in diesem Schriftstück beschriebene Ermittlung der Eindringtiefe des Gewindeelements in den Knochen hat gegenüber anderen möglichen Verfahren den großen Vorteil, dass dazu keine zusätzlichen Bauteile benötigt werden, sondern dass sie ausschließlich mit Bauteilen durchführbar ist, die zum Schneiden des Gewindes ohnehin notwendig oder vorhanden sind.

Die Ermittlung der Eindringtiefe (des fortschreitenden Eindringens) des Gewindeelements in den Knochen basiert somit vorzugsweise auf zumindest einem Parameter und / oder Messwert von zumindest einem Element der medizinischen, insbesondere dentalen, Vorrichtung zur Bestimmung der Knochenqualität, die zum Schneiden eines Gewindes mit einem durch einen motorischen Antrieb angetriebenen rotierenden Gewindeelement in den Knochen zwingend notwendig sind. Insbesondere ist die Eindringtiefe des Gewindeelements in den Knochen auf Basis von zumindest einem Parameter und / oder Messwert zumindest eines der folgenden Elemente ermittelbar: des motorischen Antriebs; einer zwischen dem motorischen Antrieb und dem Gewindeelement, insbesondere in einem Handstück, angeordneten Getriebeeinheit; des Gewindeelements zum Schneiden eines Gewindes in den Knochen.

Vorzugsweise ist der Messschaltkreis ausgebildet, zum Überwachen und / oder Ermitteln der Eindringtiefe des Gewindeelements in den Knochen Drehwinkelwerte eines, insbesondere magnetischen, Rotors des motorischen Antriebs zu verarbeiten. Die Drehwinkelwerte des Rotors sind zum Beispiel über dem Rotor zugeordnete Drehwinkelsensoren ermittelbar, zum Beispiel über Hall-Sensoren. Alternativ sind die Drehwinkelwerte bei einem sensorlosen motorischen Antrieb zum Beispiel über die Bestromung der Wicklungen oder über die kurzzeitige Abgabe hoher Spannungsimpulse an die Wicklungen eines Stators des motorischen Antriebs bestimmbar.

Es ist auch denkbar, den motorischen Antrieb mit einer konstanten Drehzahl zu betreiben, so dass eine Messung von Drehwinkelwerten des Rotors nicht notwendig ist, sondern alternativ ein fixer oder vorbestimmter Drehwinkelwert in der Steuervorrichtung, insbesondere in einem Speicher, hinterlegt ist und dem Messschaltkreis übermittelbar ist.

Vorzugsweise ist der Messschaltkreis ausgebildet, zum Überwachen und / oder Ermitteln der Eindringtiefe des Gewindeelements in den Knochen das Übersetzungsverhältnis, insbesondere einschließlich des Wirkungsgrades, einer zwischen dem motorischen Antrieb und dem Gewindeelement angeordneten Getriebeeinheit der medizinische, insbesondere dentalen, Vorrichtung zu berücksichtigen oder zu verarbeiten. Die Getriebeeinheit umfasst vorzugsweise zwei miteinander kämmende Zahnräder. Die Getriebeeinheit umfasst vorzugsweise ein Untersetzungsgetriebe. Die Getriebeeinheit ist vorzugsweise in einem Handstück angeordnet, an dem insbesondere auch eine Haltevorrichtung für das Gewindeelement vorgesehen ist.

Vorzugsweise ist das Übersetzungsverhältnis, insbesondere einschließlich des Wirkungsgrades, der Getriebeeinheit in der Steuervorrichtung, insbesondere in einem Speicher, hinterlegt und dem Messschaltkreis übermittelbar oder durch den Messschaltkreis abfragbar. Alternativ ist das Übersetzungsverhältnis, insbesondere einschließlich des Wirkungsgrades, der Getriebeeinheit in einem der Getriebeeinheit oder dem Handstück zugeordneten oder daran vorgesehenen Speicherelement hinterlegt und durch die Steuervorrichtung oder den Messschaltkreis abfragbar und dem Messschaltkreis direkt oder über einen Speicher der Steuervorrichtung übermittelbar.

Vorzugsweise ist der Messschaltkreis ausgebildet, zum Überwachen und / oder Ermitteln der Eindringtiefe des Gewindeelements in den Knochen zumindest eine Eigenschaft des Gewindeelements zu berücksichtigen, zum Beispiel die Steigung der zumindest einen Schneide, die Form der Schneide oder die Außenform des Gewindeelements. Die Schneide des Gewindeelements ist insbesondere als gewindeförmiges Schneidelement ausgebildet.

Vorzugsweise ist die zumindest eine Eigenschaft des Gewindeelements in der Steuervorrichtung, insbesondere in einem Speicher, hinterlegt und dem Messschaltkreis übermittelbar oder durch den Messschaltkreis abfragbar. Alternativ ist die zumindest eine Eigenschaft des Gewindeelements in einem dem Gewindeelement zugeordneten oder daran vorgesehenen Speicherelement hinterlegt und durch die Steuervorrichtung oder den Messschaltkreis abfragbar und dem Messschaltkreis direkt oder über einen Speicher der Steuervorrichtung übermittelbar.

Bevorzugt ist der Messschaltkreis ausgebildet, zum Überwachen und / oder Ermitteln der Eindringtiefe (des fortschreitenden Eindringens) des Gewindeelements in den Knochen mehrere oder alle der im Vorstehenden genannten Messwerte, Parameter oder Eigenschaften zu verarbeiten und / oder miteinander zu kombinieren. Insbesondere ist der Messschaltkreis ausgebildet, zum Überwachen und / oder Ermitteln der Eindringtiefe des Gewindeelements in den Knochen zumindest jeweils einen Messwert, Parameter oder eine Eigenschaft des motorischen Antriebs, der Getriebeeinheit und des Gewindeelements zu verarbeiten. Damit ist in vorteilhafter Weise eine besonders genaue Bestimmung der Eindringtiefe möglich. Besonders bevorzugt berechnet der Messschaltkreis die Eindringtiefe des Gewindeelements in den Knochen anhand folgender Gleichung:

$$D = \frac{S \times A}{T}$$

wobei D = die Eindringtiefe des Gewindeelements in den Knochen, S = die Steigung der zumindest einen Schneide des Gewindeelements, A = der Drehwinkel des motorischen Antriebs und T = das Übersetzungsverhältnis der Getriebeeinheit ist.

Vorzugsweise ist in der Steuervorrichtung zumindest eine vorbestimmte Eindringtiefe gespeichert, zum Beispiel 6 mm oder 8 mm. Alternativ sind in der Steuervorrichtung mehrere vorbestimmte Eindringtiefen gespeichert, die über ein Bedienelement der Vorrichtung zur Bestimmung der Knochenqualität oder der Steuervorrichtung durch den Anwender auswählbar sind. Besonders bevorzugt ist die Steuervorrichtung, zum Beispiel der Messschaltkreis oder eine andere Schaltung, ausgebildet, die überwachte und / oder ermittelte Eindringtiefe des Gewindeelements mit der zumindest einen vorbestimmten Eindringtiefe zu vergleichen, um bei Erreichen oder Überschreiten der zumindest einen vorbestimmten Eindringtiefe den motorischen Antrieb zu stoppen und / oder seine Drehrichtung zu ändern.

Die Steuervorrichtung, insbesondere der Messschaltkreis, ist ausgebildet, mit der Überwachung und / oder Ermittlung oder mit einer Aufzeichnung oder Speicherung der Eindringtiefe (des fortschreitenden Eindringens) des Gewindeelements in den Knochen erst nach Erreichen oder Überschreiten eines vorbestimmten Motorstromschwellwertes oder Drehmomentschwellwertes zu beginnen. Damit wird eine zuverlässige Ermittlung der Eindringtiefe des Gewindeelements garantiert, da durch das Erreichen oder Überschreiten des vorbestimmten Motorstromschwellwerts / Drehmomentschwellwertes gewährleistet ist, dass das Gewindeelement tatsächlich ein Gewinde schneidet und / oder in den Knochen vordringt. Der vorbestimmte Motorstromschwellwert / Drehmomentschwellwert ist vorzugsweise in der Steuervorrichtung, insbesondere in einem Speicher, hinterlegt. Der vorbestimmte Motorstromschwellwert / Drehmomentschwellwert ist vorzugsweise für einen Anwender nicht veränderbar. Die

Steuervorrichtung, insbesondere der Messschaltkreis, sind vorzugsweise ausgebildet, bis zum Erreichen des vorbestimmten Motorstromschwellwerts / Drehmomentschwellwerts wiederholt den aktuellen, von dem Messschaltkreis bestimmten Motorstromwert / Drehmomentwert mit dem vorbestimmten Motorstromschwellwert / Drehmomentschwellwert zu vergleichen.

Vorzugsweise umfasst die medizinische, insbesondere dentale oder dentalchirurgische, Vorrichtung eine mit der zweiten Kontaktvorrichtung kommunikativ verbundene Anzeigeeinheit, welche den Zusammenhang zwischen den bestimmten Motorstromwerten, den zu den Motorstromwerten korrelierenden oder im Wesentlichen proportionalen Drehmomentwerten und / oder davon abgeleiteten Parametern, insbesondere der Qualität eines Knochens, und der Eindringtiefe wiedergibt, insbesondere grafisch darstellt. Die Anzeigeeinheit umfasst insbesondere einen Bildschirm oder einen Monitor. Die Anzeigeeinheit ist wahlweise als separate, mit der Steuervorrichtung operativ, insbesondere drahtlos über die im Vorstehenden beschriebene drahtlose zweite Kontaktvorrichtung, verbundene Einheit oder als integraler Teil der Steuervorrichtung ausgebildet.

Die Anzeigeeinheit stellt den Zusammenhang zwischen den bestimmten Motorstromwerten, den zu den Motorstromwerten korrelierenden oder im Wesentlichen proportionalen Drehmomentwerten und / oder davon abgeleiteten Parametern, insbesondere der Qualität eines Knochens, und der Eindringtiefe, insbesondere das qualitative Tiefenprofil des Knochens, vorzugsweise grafisch in Form eines Diagramms oder einer schematischen Darstellung einer, zum Beispiel schichtweise aufgebauten, Knochenstruktur dar.

Das Diagramm oder die schematische Darstellung sind vorzugsweise derart ausgebildet, dass jedem (einzelnen) Wert der Eindringtiefe ein Motorstromwert, zum Motorstromwert korrelierender oder im Wesentlichen proportionaler Drehmomentwert und / oder ein davon abgeleiteter Parameter, insbesondere eine Qualität des Knochens, zugeordnet ist. Der jedem einzelnen Wert der Eindringtiefe zugeordnete Motorstromwert, Drehmomentwert und / oder einen davon abgeleiteter Parameter, insbesondere die Qualität des Knochens, kann entweder ebenfalls ein Einzelwert sein oder gemittelte oder zusammengefasste Werte mehrerer bestimmter Motorstromwerte, Drehmomentwerte und / oder davon abgeleiteter Parameter, insbesondere der Qualität eines Knochens, umfassen. Die zusammengefassten Werte umfassen zum Beispiel unterschiedliche Klassen von Knochenqualitäten, zum Beispiel Knochenqualität Klasse 1, Knochenqualität Klasse 2, Knochenqualität Klasse 3, usw.

Demgemäß ist die Anzeigeeinheit vorzugsweise ausgebildet, auf Basis der Messsignale des Messschaltkreises ein Diagramm oder eine schematische Darstellung anzuzeigen, bei dem / der jedem (einzelnen) Wert der Eindringtiefe ein einzelner Wert des bestimmten Motorstroms, eines dazu korrelierenden oder im Wesentlichen proportionalen Drehmomentwerts und / oder eines davon abgeleiteten Parameters, insbesondere die Qualität des Knochens, zugeordnet ist. Alternativ ist die Anzeigeeinheit ausgebildet, auf Basis der Messsignale des Messschaltkreises ein Diagramm oder eine schematische Darstellung anzuzeigen, bei dem / der (einzelnen) Werten der Eindringtiefe gemittelte oder zusammengefasste Werte mehrerer bestimmter Motorstromwerte, Drehmomentwerte und / oder davon abgeleiteter Parameter, insbesondere der Qualität eines Knochens, zugeordnet sind.

Das von der Anzeigeeinheit dargestellte Diagramm ist vorzugsweise als zweidimensionales Diagramm ausgebildet, an dessen Abszisse insbesondere die Eindringtiefe (in mm) und an dessen Ordinate der Motorstromwert, der zum Motorstromwert korrelierende oder im Wesentlichen proportionale Drehmomentwert und / oder ein davon abgeleiteter Parameter, insbesondere der Qualität eines Knochens, aufgetragen ist. Das Diagramm ist vorzugsweise als Liniendiagramm ausgebildet, selbstverständlich kann es jedoch auch eine beliebige andere Diagrammart aufweisen, zum Beispiel ein Säulendiagramm, ein Balkendiagramm oder ein Punktediagramm.

Die von der Anzeigeeinheit dargestellte schematische Darstellung einer, insbesondere schichtweise aufgebauten, Knochenstruktur ist vorzugsweise als zweidimensionale Darstellung ausgebildet, an deren Ordinate insbesondere die Eindringtiefe (in mm) aufgetragen ist. Vorzugsweise erstrecken sich von der Ordinate horizontale Balken oder Streifen, die gemittelte oder zusammengefasste Werte mehrerer bestimmter Motorstromwerte, zu den Motorstromwerten korrelierender oder im Wesentlichen proportionaler Drehmomentwerte und / oder davon abgeleiteter Parameter, insbesondere die Qualität des Knochens, darstellen. Die horizontalen Balken oder Streifen weisen besonders bevorzugt unterschiedliche Markierungen oder Farben auf.

Vorzugsweise weist die medizinische, insbesondere dentale, Vorrichtung zur Bestimmung der Knochenqualität des Weiteren einen motorischen Antrieb und ein mit dem Gewindeelement verbindbares oder verbundenes Handgriffelement auf, das mit dem motorischen Antrieb kuppelbar ist oder den motorischen Antrieb umfasst. Der motorische Antrieb ist vorzugsweise als elektromotorischer Antrieb ausgebildet, zum Beispiel als bürstenloser E-Motor. Das Handstück ist vorzugsweise als gewinkeltes Handstück oder Winkelstück ausgebildet. An dem Handstück, insbesondere an dessen Vorderende oder Kopfteil, ist vorzugsweise eine Haltevorrichtung für das Gewindeelement vorgesehen, insbesondere eine lösbare Spannvorrichtung. An dem Handstück, insbesondere an dessen Vorderende oder Kopfteil, ist vorzugsweise eine Beleuchtungsvorrichtung zum Beleuchten der Präparationsstelle mit sichtbarem Licht vorgesehen.

Vorzugsweise umfasst die medizinische, insbesondere dentale, Vorrichtung zur Bestimmung der Knochenqualität, insbesondere die Steuervorrichtung, zumindest ein Speicherelement auf, in dem zumindest die aktuellen Messwerte der bestimmten Motorstromwerte, der zu den Motorstromwerten korrelierenden oder im Wesentlichen proportionalen Drehmomentwerte oder davon abgeleiteter Parameter, insbesondere der Qualität des Knochens, und der Eindringtiefe speicherbar sind.

Vorzugsweise weist die Steuervorrichtung eine Auswerteschaltung auf, die ausgebildet ist, die von dem Messschaltkreis bestimmten Motorstromwerte, die zu den Motorstromwerten korrelierenden oder im Wesentlichen proportionalen Drehmomentwerte oder davon abgeleitete Parameter mit Vergleichswerten zur Bestimmung der Qualität eines Knochens zu vergleichen und / oder zu Knochenqualitäts-Klassen zusammenzufassen und / oder Knochenqualitäts-Klassen zuzuordnen. Vorzugsweise ist die Auswerteschaltung mit dem Messschaltkreis zum Empfangen der Messsignale zumindest kommunikativ verbunden oder als Teil des Messschaltkreises ausgebildet. Vorzugsweise ist die Auswerteschaltung mit der Anzeigeeinheit verbunden, so dass die Anzeigeeinheit von der Auswerteschaltung bearbeitete, verglichene oder zusammengefasste Daten, insbesondere KnochenqualitätsKlassen, darstellen kann.

Vorzugsweise ist die Steuervorrichtung, zum Beispiel der Messschaltkreis oder die Auswerteschaltung, des Weiteren ausgebildet, den Verlauf und / oder die integrierten Daten der von dem motorischen Antrieb benötigten oder aufgewendeten Energie oder Leistung während des Schneidens des Gewindes in einen Knochen zu ermitteln und ein entsprechendes Anzeigesignal über die zweite Kontaktvorrichtung an die Anzeigeeinheit zu übertragen, so dass die Anzeigeeinheit auf Basis des übertragenen Anzeigesignals die während des Schneidens des Gewindes benötigte oder aufgewendete Energie oder Leistung wiedergibt. Vorzugsweise berechnet die Steuervorrichtung die benötigte oder aufgewendete Energie oder Leistung über den Verlauf oder die Integration der bestimmten Motorstrom- oder der davon abgeleiteten Drehmomentwerte, der maximalen Eindringtiefe und / oder des Drehwinkels, der benötigten Zeit bis zum Erreichen der maximalen Eindringtiefe und gegebenenfalls der zu bestimmenden Drehzahl des rotierenden Gewindeelements.

Vorzugsweise umfasst das rotierende Gewindeelement einen Gewindeschneider oder ein Implantat, insbesondere ein selbstschneidendes Implantat. Das rotierende Gewindeelement umfasst zumindest ein sich wendelförmig oder spiralförmig um einen Körper des Gewindeelements erstreckendes Schneidelement, insbesondere ein Knochen schneidendes Schneidgewinde.

Die Erfindung wird nachfolgend anhand bevorzugter Ausführungsbeispiele und Bezug nehmend auf die beigefügten Zeichnungen erläutert. Es zeigt die

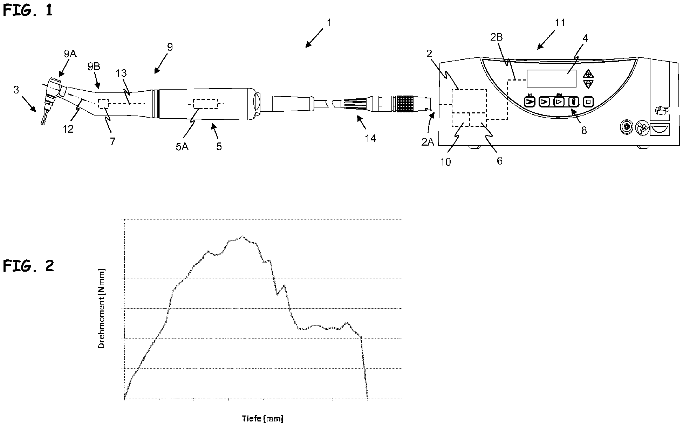

Figur 1 eine medizinische, insbesondere dentale oder dentalchirurgische, Vorrichtung zur Bestimmung der Qualität eines Knochens mit einem Handgriffelement, einem Gewindeelement und einer Bedien- und/oder Steuerkonsole.

Figur 2 ein erstes Ausführungsbeispiel eines von der Anzeigeeinheit anzeigbaren Diagramms, das den Zusammenhang zwischen den bestimmten Drehmomentwerten, die korrelierend oder im Wesentlichen proportional zu den bestimmten Motorstromwerten sind, und der Eindringtiefe, insbesondere das qualitative Tiefenprofil des Knochens, wiedergibt.

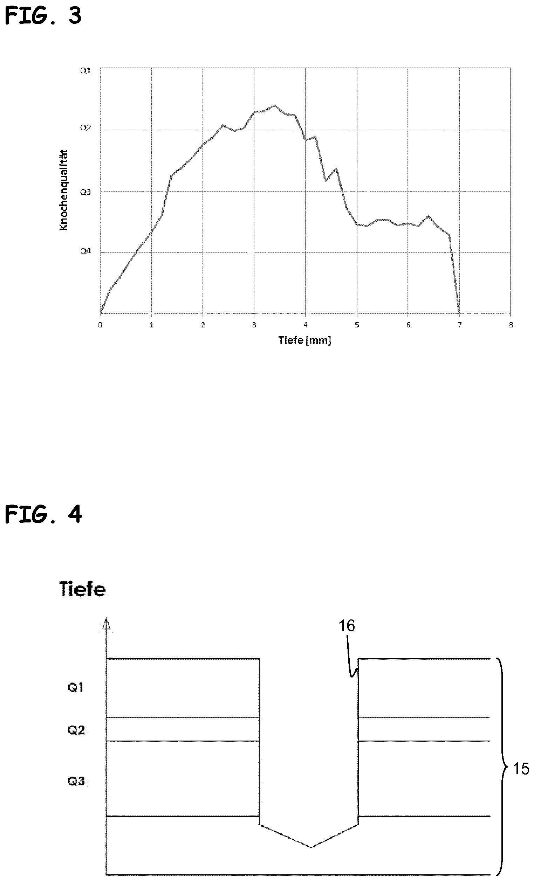

Figur 3 ein zweites Ausführungsbeispiel eines von der Anzeigeeinheit anzeigbaren Diagramms, das den Zusammenhang zwischen der Knochenqualität, basierend auf den bestimmten Motorstromwerten, und der Eindringtiefe, insbesondere das qualitative Tiefenprofil des Knochens, wiedergibt.

Figur 4 ein Ausführungsbeispiel einer von der Anzeigeeinheit anzeigbaren Darstellung, die den Zusammenhang zwischen der Knochenqualität, basierend auf den bestimmten Motorstromwerten, und der Eindringtiefe, insbesondere das qualitative Tiefenprofil des Knochens, an einer schematisch dargestellten Knochenstruktur wiedergibt.

Die in der Figur 1 dargestellte medizinische, insbesondere dentale oder dentalchirurgische, Vorrichtung 1 zur Bestimmung der Qualität eines Knochens, insbesondere eines Kieferknochens, umfasst ein Handgriffelement 9, ein rotierendes Gewindeelement 3 mit zumindest einer Schneide, einen motorischen, insbesondere elektromotorischen, Antrieb 5 und eine Steuervorrichtung 2 mit einem Messschaltkreis 6.

Das als Winkelstück ausgebildete Handgriffelement 9 umfasst einen Kopfabschnitt 9A und einen Griffabschnitt 9B mit einer Längsachse 12. Das rotierende Gewindeelement 3 ist derart an dem Kopfabschnitt 9A befestigt, insbesondere in einer lösbaren Aufnahme oder Haltevorrichtung, dass es gewinkelt zu der Längsachse 12 des Griffabschnitts 9B angeordnet ist.

In dem Handgriffelement 9 sind zumindest eine oder mehrere, insbesondere rotierbare, Antriebswellen 13 und eine Getriebeeinheit 7, insbesondere mit einem Untersetzungsgetriebe, vorgesehen. Die zumindest eine Antriebswelle 13 und die Getriebeeinheit 7 sind insbesondere miteinander und mit dem motorischen Antrieb 5 verbunden, so dass eine rotierende Bewegung des motorischen Antriebs 5 über die Antriebswelle 13 und die Getriebeeinheit 7 auf das rotierende oder in Rotation versetzbare Gewindeelement 3 übertragbar ist.

Der (elektro)motorische Antrieb 5 oder Elektromotor ist zum Antreiben des rotierbaren Gewindeelements 3, der zumindest einen rotierbaren Antriebswelle 13 und der Getriebeeinheit 7 vorgesehen. Der motorische Antrieb 5 ist vorzugsweise ein eigenständiges Bauteil, das lösbar mit dem Handgriffelement 9 und/oder der zumindest einen rotierbaren Antriebswelle 13 verbindbar ist, zum Beispiel über eine Kupplungsvorrichtung. Alternativ ist der motorische Antrieb 5 als Teil des Handgriffelements 9 ausgebildet. Der (elektro)motorische Antrieb 5 umfasst insbesondere einen Stator, einen relativ dazu bewegbaren Rotor 5A, der zumindest ein Magnetelement aufweist, und zumindest eine Motorwelle, die mit der Antriebswelle 13 des Handgriffelements 9 kuppelbar oder verbunden ist.

Bevorzugt sind der motorische Antrieb 5 und das Handgriffelement 9 über einen Versorgungsschlauch 14 mit einer Bedien- und/oder Steuerkonsole 11 verbunden. In dem Versorgungsschlauch 14 ist zumindest eine Versorgungs- oder Steuerleitung oder sind insbesondere mehrere elektrische Leiter zum Übertragen von Antriebsenergie für den motorischen Antrieb 5 und zur Übertragung von Steuer,- Regel- oder Messsignalen, vorzugsweise auch zur Übertragung von Energie für eine Beleuchtungsvorrichtung, angeordnet. Der Versorgungsschlauch 14 ist zum Beispiel lösbar mit der Bedien- und/oder Steuerkonsole 11 und lösbar oder für den Anwender unlösbar mit dem motorischen Antrieb 5 verbunden.

Wie aus der Figur 1 zu erkennen ist, ist in der Bedien- und/oder Steuerkonsole 11 eine Steuervorrichtung 2 vorgesehen, die zumindest einen Messschaltkreis 6 und vorzugsweise auch eine Auswerteschaltung 10 umfasst. Neben diesen beiden Schaltungen 6, 10 sind vorzugsweise zumindest eine weitere Schaltung und / oder zumindest ein Element der Vorrichtung 1 in der Konsole 11 vorgesehen, zum Beispiel: eine Steuer- oder Regelschaltung des motorischen Antriebs 5; eine Komparatorschaltung, zum Beispiel als Teil der Auswerteschaltung 10, die ausgebildet ist, die von dem Messschaltkreis bestimmten Motorstromwerte, die zu den Motorstromwerten korrelierenden oder im Wesentlichen proportionalen Drehmomentwerte oder davon abgeleitete Parameter mit Vergleichswerten zur Bestimmung der Qualität eines Knochens zu vergleichen; eine Anzeigeeinheit 4; zumindest ein Bedien- oder Stellelement 8 zum Einstellen oder Auswählen von Betriebsparametern; ein, insbesondere mit dem zumindest einen Bedien- oder Stellelement 8 verbundener, Schaltkreis zum Bedienen oder Einstellen von Betriebsparametern für die Vorrichtung 1 und / oder für den elektromotorischen Antrieb 5; eine Speichereinheit, in welcher Messsignale, Motorstromwerte, zu den Motorstromwerten korrelierende oder im Wesentlichen proportionale Drehmomentwerte, davon abgeleitete Parameter, insbesondere die Qualität eines Knochens, und / oder Werte der Eindringtiefe speicherbar sind.

Alternativ ist der Messschaltkreis 6, vorzugsweise auch die Auswerteschaltung 10 und zumindest ein(es) der weiteren im Vorstehenden genannten Schaltungen oder Elemente, an dem Handgriffelement 9 und / oder an dem motorischen Antrieb 5 vorgesehen, so dass die Vorrichtung 1 zur Bestimmung der Knochenqualität als kabelloses Handgriffelement ausgebildet ist.

Die Steuervorrichtung 2, insbesondere der Messschaltkreis 6, ist operativ, zum Beispiel über elektrische Leitungen, mit einer ersten elektrischen Kontaktvorrichtung 2A und einer zweiten, vorzugsweise ebenfalls elektrischen, Kontaktvorrichtung 2B verbunden. Die erste elektrische Kontaktvorrichtung 2A verbindet die Steuervorrichtung 2 oder den Messschaltkreis 6 mit dem motorischen Antrieb 5, insbesondere über den Versorgungsschlauch 14 und / oder zumindest eine Versorgungs- oder Steuerleitung. Die zweite elektrische Kontaktvorrichtung 2B verbindet die Steuervorrichtung 2 oder den Messschaltkreis 6 mit der Anzeigeeinheit 4. Die Kontaktvorrichtungen 2A, 2B sind vorzugsweise Teil der Bedien- und/oder Steuerkonsole 11.

Der Messschaltkreis 6 ist ausgebildet, über die erste elektrische Kontaktvorrichtung 2A Werte des Motorstroms zu bestimmen, mit dem der motorische Antrieb 5 zum rotierenden Antreiben des mit dem motorischen Antrieb 5 verbindbaren Gewindeelements 3 versorgt wird. Da die Motorstromwerte korrelierend oder im Wesentlichen proportional zu übertragenen Drehmomentwerten des motorischen Antriebs 5 sind und da die Motorstromwerte oder Drehmomentwerte ein Maß für die Qualität eines Knochens sind, ist der Messschaltkreis 6 somit ausgebildet, die Knochenqualität zu bestimmen, insbesondere während des Antriebs des rotierenden Gewindeelements 3 oder des Vordringens des rotierenden Gewindeelements 3 in den Knochen.

Der Messschaltkreis 6 ist des Weiteren ausgebildet, die Eindringtiefe (d.h. das Fortschreiten des Eindringens) des Gewindeelements 3 in den Knochen zu überwachen und / oder zu ermitteln. Dazu verarbeitet der Messschaltkreis 6 zum Beispiel Drehwinkelwerte der Rotors 5A des motorischen Antriebs 5 und / oder das Übersetzungsverhältnis, insbesondere einschließlich des Wirkungsgrades, der Getriebeeinheit 7 des Handgriffelements 9 und / oder zumindest eine Eigenschaft des rotierenden Gewindeelements 3, zum Beispiel die Steigung der zumindest einen Schneide, die Form der Schneide oder die Außenform des Gewindeelements 3.

Schließlich ist der Messschaltkreis 6 ausgebildet, Messsignale zu erzeugen, welche die bestimmten Motorstromwerte, die zu den Motorstromwerten korrelierenden oder im Wesentlichen proportionalen Drehmomentwerte oder davon abgeleitete Parameter mit der Eindringtiefe in Bezug setzen, und diese Messsignale über die zweite Kontaktvorrichtung 2B an die Anzeigeeinheit 4 zu übertragen.

Die Anzeigeeinheit 4 ist ausgebildet, auf Basis der von dem Messschaltkreis 6 übertragenen Messsignale den Zusammenhang zwischen den bestimmten Motorstromwerten, den zu den Motorstromwerten korrelierenden oder im Wesentlichen proportionalen Drehmomentwerten und / oder den davon abgeleiteten Parametern, insbesondere der Qualität eines Knochens, und der Eindringtiefe, insbesondere das qualitative Tiefenprofil des Knochens, wiederzugeben. Die Anzeigeeinheit 4 umfasst insbesondere einen in das Gehäuse der Bedien- und/oder Steuerkonsole 11 integrierten Bildschirm, auf dem Diagramme oder grafische Darstellungen anzeigbar sind. Die Anzeigeeinheit 4 ist vorzugsweise auch zur Anzeige von Betriebsparametern der Vorrichtung 1 und / oder von einem durch das Bedien- oder Stellelement 8 veränderbaren Parameter ausgebildet.

In der Steuervorrichtung 2, insbesondere in einem Speicherelement, ist vorzugsweise zumindest eine vorbestimmte Eindringtiefe des rotierenden Gewindeelements 3 in den Knochen gespeichert. Die Steuervorrichtung 2 ist vorzugsweise ausgebildet, die überwachte und / oder ermittelte Eindringtiefe des Gewindeelements 3 mit der zumindest einen vorbestimmten Eindringtiefe zu vergleichen und bei Erreichen oder Überschreiten der zumindest einen vorbestimmten Eindringtiefe den motorischen Antrieb 5 zu stoppen und / oder seine Drehrichtung zu ändern.

Die Auswerteschaltung 10 als Teil der Steuervorrichtung 2 oder des Messschaltkreises 6 ist ausgebildet, die von dem Messschaltkreis 6 bestimmten Motorstromwerte, die zu den Motorstromwerten korrelierenden oder im Wesentlichen proportionalen Drehmomentwerte oder davon abgeleitete Parameter mit Vergleichswerten zur Bestimmung der Qualität eines Knochens zu vergleichen und / oder zu KnochenqualitätsKlassen zusammenzufassen und / oder Knochenqualitäts-Klassen zuzuordnen. Die Auswerteschaltung 10 ist mit der Anzeigeeinheit 4 kommunikativ verbunden, so dass die durch die Auswerteschaltung 10 verglichenen Werte und / oder die Knochenqualitäten und / oder die Knochenqualitäts-Klassen durch die oder auf der Anzeigeeinheit 4 darstellbar sind.

Die im Vorstehenden genannte Steuer- oder Regelschaltung des motorischen Antriebs oder Elektromotors 5 ist ausgebildet, den motorischen Antrieb 5 mittels elektrischer Signale über den Versorgungsschlauch 14 oder die zumindest eine Versorgungs- oder Steuerleitung zu steuern oder zu regeln. Gegebenenfalls ist die Steuer- oder Regelschaltung auch dazu vorgesehen, den motorischen Antrieb 5 mit einem vorbestimmten oder fixen Drehzahlwert zu betreiben.

Vorzugsweise sind alle im Vorstehenden genannten Schaltkreise und Schaltungen 6, 10 als elektronische Schaltungen, insbesondere als Teil eines Mikroprozessors, ausgebildet.

In der Figur 2 ist ein von der Anzeigeeinheit 4 anzeigbares, zweidimensionales Diagramm dargestellt, das den Zusammenhang zwischen den bestimmten Drehmomentwerten, die korrelierend oder im Wesentlichen proportional zu den bestimmten Motorstromwerten sind, und der Eindringtiefe wiedergibt. An der Abszisse des Diagramms ist die Eindringtiefe (vorzugsweise in mm) und an der Ordinate sind die Drehmomentwerte (vorzugsweise in Nmm oder Ncm) aufgetragen. Das Diagramm ist als Liniendiagramm ausgebildet, wobei jedem einzelnen Wert der Eindringtiefe ein (eigener) Drehmomentwert zugeordnet ist. Damit kann der Anwender für jeden beliebigen Wert der Eindringtiefe den von dem Messschaltkreis 6 bestimmten Drehmomentwert ablesen und / oder daraus die Qualität des Knochens einschätzen, da, wie im Vorstehenden bereits beschrieben, die von dem Messschaltkreis 6 bestimmten Motorstromwerte oder die dazu korrelierenden oder im Wesentlichen proportionalen Drehmomentwerte ein Maß für die Knochenqualität sind (je höher die Motorstromwerte oder die Drehmomentwerte sind, umso höher oder besser ist die Knochenqualität).

In der Figur 3 ist ein von der Anzeigeeinheit 4 anzeigbares, zweidimensionales Diagramm dargestellt, das den Zusammenhang zwischen der Knochenqualität, die von den durch den Messschaltkreis 6 bestimmten Drehmomentwerten oder den dazu korrelierenden oder im Wesentlichen proportionalen bestimmten Motorstromwerten herleitbar ist, und der Eindringtiefe wiedergibt. An der Abszisse des Diagramms ist die Eindringtiefe (vorzugsweise in mm) und an der Ordinate ist die Knochenqualität aufgetragen. Das Diagramm ist wiederum als Liniendiagramm ausgebildet, wobei jedem einzelnen Wert der Eindringtiefe eine Knochenqualitätsklasse zugeordnet ist. Damit kann der Anwender für jeden beliebigen Wert der Eindringtiefe die Knochenqualität oder Knochenqualitätsklasse ablesen.

Das Diagramm der Figur 3 umfasst beispielhaft vier Knochenqualitätsklasse Q1 - Q4, wobei jedoch selbstverständlich auch mehr oder weniger Knochenqualitätsklassen denkbar sind, zum Beispiel zwei, drei, fünf, sechs oder mehr Knochenqualitätsklassen. Jede Knochenqualitätsklasse umfasst oder repräsentiert einen vordefinierten Bereich von durch den Messschaltkreis 6 bestimmten Motorstromwerten oder dazu korrelierenden oder im Wesentlichen proportionalen bestimmten Drehmomentwerten. So umfasst zum Beispiel die Knochenqualitätsklasse Q4 Drehmomentwerte von 8,00 Ncm - 6,00 Ncm, die Knochenqualitätsklasse Q3 Drehmomentwerte von 5,99 Ncm - 4,00 Ncm, usw. Demgemäß ist die in dem Diagramm der Figur 3 dargestellte Knochenqualität in geringer Eindringtiefe und bei weit fortgeschrittener Eindringtiefe hoch, das heißt der Knochen ist hart, und im Bereich der mittleren Eindringtiefe ist die Knochenqualität gering, das heißt der Knochen ist weich.

Die Figur 4 zeigt ein Ausführungsbeispiel einer von der Anzeigeeinheit 4 anzeigbaren, insbesondere zweidimensionalen, Darstellung, die den Zusammenhang zwischen der Knochenqualität, basierend auf den durch den Messschaltkreis 6 bestimmten Motorstromwerten, und der Eindringtiefe an einer schematisch dargestellten Knochenstruktur 15 wiedergibt. In der Knochenstruktur 15 ist, ebenfalls schematisch, eine Bohrung 16 dargestellt, die insbesondere jene Bohrung repräsentiert, in die das Gewindeelement 3 der medizinischen, insbesondere dentalen oder dentalchirurgischen, Vorrichtung 1 ein Gewinde schneidet.

An der Ordinate der Darstellung der Figur 4 ist die Eindringtiefe (in mm) aufgetragen. Von der Ordinate erstrecken sich horizontale Balken oder Streifen, welche die Qualität des Knochens in Form von Knochenqualitätsklassen darstellen. Auch bei dieser Darstellung ist jedem einzelnen Wert der Eindringtiefe eine Knochenqualitätsklasse zugeordnet.

Jede Knochenqualitätsklasse umfasst oder repräsentiert einen vordefinierten Bereich von durch den Messschaltkreis 6 bestimmten Motorstromwerten oder dazu korrelierenden oder im Wesentlichen proportionalen Drehmomentwerten, so wie dies in Verbindung mit dem Diagramm der Figur 3 beschrieben ist. Die horizontalen Balken oder Streifen weisen besonders bevorzugt unterschiedliche Markierungen oder Farben auf, insbesondere ist jeder Knochenqualitätsklasse eine eigene Markierung oder Farbe zugeordnet. Die Darstellung der Figur 4 umfasst beispielhaft drei Knochenqualitätsklasse Q1 - Q3, wobei jedoch selbstverständlich wiederum mehr oder weniger Knochenqualitätsklassen denkbar sind, zum Beispiel zwei, vier, fünf, sechs oder mehr Knochenqualitätsklassen. Die Darstellung gemäß der Figur 4 liefert dem Anwender einen besonders schnell aufnehmbaren und gut verständlichen Überblick über den Verlauf der Knochenqualität entlang der Eindringtiefe.

Die Erfindung ist nicht auf die beschriebenen Ausführungsbeispiele beschränkt, sondern umfasst alle Ausführungen, die das prinzipielle, sinngemäße Funktionsprinzip der Erfindung gemäß den Ansprüchen anwenden oder beinhalten. Des Weiteren sind alle Merkmale aller beschriebenen und dargestellten Ausführungsbeispiele miteinander kombinierbar.

## Patentansprüche

1. Medizinische, insbesondere dentale, Vorrichtung (1) zur Bestimmung der Qualität eines Knochens durch Schneiden eines Gewindes in den Knochen mittels einem rotierenden Gewindeelement (3) mit zumindest einer Schneide, wobei die medizinische, insbesondere dentale, Vorrichtung (1) eine Steuervorrichtung (2) umfasst, welche eine erste elektrische Kontaktvorrichtung (2A) zur Verbindung mit einem motorischen Antrieb (5), eine zweite Kontaktvorrichtung (2B) zur Verbindung mit einer Anzeigeeinheit (4) und einen Messschaltkreis (6), der operativ mit der ersten elektrischen Kontaktvorrichtung (2A) und der zweiten Kontaktvorrichtung (2B) verbunden ist, aufweist, wobei der Messschaltkreis (6) ausgebildet ist:

   (i) über die erste elektrische Kontaktvorrichtung (2A) Werte des Motorstroms zu bestimmen, mit dem der motorische Antrieb (5) zum rotierenden Antreiben des mit dem motorischen Antrieb (5) verbindbaren Gewindeelements (3) versorgt wird, wobei die Motorstromwerte korrelierend oder im Wesentlichen proportional zu übertragenen Drehmomentwerten des motorischen Antriebs (5) sind und wobei die Motorstromwerte oder Drehmomentwerte ein Maß für die Qualität eines Knochens sind,
   (ii) die Eindringtiefe des Gewindeelements (3) in den Knochen zu überwachen und / oder zu ermitteln und
   (iii) Messsignale zu erzeugen, welche die bestimmten Motorstromwerte, die zu den Motorstromwerten korrelierenden oder im Wesentlichen proportionalen Drehmomentwerte oder die von den bestimmten Motorstromwerten oder den Drehmomentwerten abgeleitete Qualität eines Knochens mit der Eindringtiefe in Bezug setzen, und diese Messsignale über die zweite Kontaktvorrichtung (2B) an die Anzeigeeinheit (4) zu übertragen, so dass die Anzeigeeinheit (4) auf Basis der übertragenen Messsignale den Zusammenhang zwischen der Eindringtiefe und zumindest einem der folgenden Parameter, nämlich den bestimmten Motorstromwerten, den zu den Motorstromwerten korrelierenden oder im Wesentlichen proportionalen Drehmomentwerten oder der Qualität eines Knochens wiedergibt,

   **dadurch gekennzeichnet, dass**
   die Steuervorrichtung (2) ausgebildet ist, mit der Überwachung, Ermittlung oder mit einer Aufzeichnung der Eindringtiefe oder des fortschreitenden Eindringens des Gewindeelements (3) in den Knochen erst nach Erreichen oder Überschreiten eines vorbestimmten Motorstromschwellwertes oder Drehmomentschwellwertes zu beginnen.

2. Medizinische, insbesondere dentale, Vorrichtung (1) nach Anspruch 1, **dadurch gekennzeichnet, dass** der Messschaltkreis (6) ausgebildet ist, zum Überwachen und / oder Ermitteln der Eindringtiefe des Gewindeelements (3) in den Knochen Drehwinkelwerte eines Rotors (5A) des motorischen Antriebs (5) zu verarbeiten.

3. Medizinische, insbesondere dentale, Vorrichtung (1) nach Anspruch 1 oder 2, **dadurch gekennzeichnet, dass**
   der Messschaltkreis (6) ausgebildet ist, zum Überwachen und / oder Ermitteln der Eindringtiefe des Gewindeelements (3) in den Knochen das Übersetzungsverhältnis, insbesondere einschließlich des Wirkungsgrades, einer zwischen dem motorischen Antrieb (5) und dem Gewindeelement (3) angeordneten Getriebeeinheit (7) der medizinische, insbesondere dentalen, Vorrichtung (1) zu berücksichtigen.

4. Medizinische, insbesondere dentale, Vorrichtung (1) nach einem der vorstehenden Ansprüche, **dadurch gekennzeichnet, dass**
   der Messschaltkreis (6) ausgebildet ist, zum Überwachen und / oder Ermitteln der Eindringtiefe des Gewindeelements (3) in den Knochen zumindest eine Eigenschaft des Gewindeelements (3) zu berücksichtigen, zum Beispiel die Steigung der zumindest einen Schneide, die Form der Schneide oder die Außenform des Gewindeelements (3).

5. Medizinische, insbesondere dentale, Vorrichtung (1) nach einem der vorstehenden Ansprüche, **dadurch gekennzeichnet, dass**
   in der Steuervorrichtung (2) zumindest eine vorbestimmte Eindringtiefe gespeichert ist.

6. Medizinische, insbesondere dentale, Vorrichtung (1) nach einem der vorstehenden Ansprüche, **dadurch gekennzeichnet, dass**
   in der Steuervorrichtung (2) mehrere vorbestimmte Eindringtiefen gespeichert sind, die über ein Stellelement (8) durch den Anwender auswählbar sind.

7. Medizinische, insbesondere dentale, Vorrichtung (1) nach Anspruch 5 oder 6, **dadurch gekennzeichnet, dass**
   die Steuervorrichtung (2) ausgebildet ist, die überwachte und / oder ermittelte Eindringtiefe des Gewindeelements (3) mit der zumindest einen vorbestimmten Eindringtiefe zu vergleichen und bei Erreichen oder Überschreiten der zumindest einen vorbestimmten Eindringtiefe den motorischen Antrieb

(5) zu stoppen und / oder seine Drehrichtung zu ändern.

8. Medizinische, insbesondere dentale, Vorrichtung (1) nach einem der vorstehenden Ansprüche, **dadurch gekennzeichnet, dass**
die Steuervorrichtung (2) ausgebildet ist, bis zum Erreichen des vorbestimmten Motorstromschwellwerts oder Drehmomentschwellwerts wiederholt den aktuellen, von dem Messschaltkreis (6) bestimmten Motorstromwert oder Drehmomentwert mit dem vorbestimmten Motorstromschwellwert oder Drehmomentschwellwert zu vergleichen.

9. Medizinische, insbesondere dentale, Vorrichtung (1) nach einem der vorstehenden Ansprüche, **gekennzeichnet durch**
eine mit der zweiten Kontaktvorrichtung (2B) kommunikativ verbundene Anzeigeeinheit (4), welche den Zusammenhang zwischen der Eindringtiefe und zumindest einem der folgenden Parameter, nämlich den bestimmten Motorstromwerten, den zu den Motorstromwerten korrelierenden oder im Wesentlichen proportionalen Drehmomentwerten oder der Qualität eines Knochens wiedergibt.

10. Medizinische, insbesondere dentale, Vorrichtung (1) nach Anspruch 9, **dadurch gekennzeichnet, dass** die Anzeigeeinheit (4) den Zusammenhang zwischen der Eindringtiefe und zumindest einem der folgenden Parameter, nämlich den bestimmten Motorstromwerten, den zu den Motorstromwerten korrelierenden oder im Wesentlichen proportionalen Drehmomentwerten oder der Qualität eines Knochens grafisch darstellt, zum Beispiel in Form eines Diagramms oder einer schematischen Darstellung einer Knochenstruktur.

11. Medizinische, insbesondere dentale, Vorrichtung (1) nach Anspruch 10, **dadurch gekennzeichnet, dass** die Anzeigeeinheit (4) ausgebildet ist, auf Basis der Messsignale des Messschaltkreises (6) ein Diagramm oder eine schematische Darstellung einer Knochenstruktur anzuzeigen, bei dem / der jedem Wert der Eindringtiefe ein Wert des bestimmten Motorstroms, eines dazu korrelierenden oder im Wesentlichen proportionalen Drehmomentwerts oder eine Qualität des Knochens zugeordnet ist, oder dass die Anzeigeeinheit (4) ausgebildet ist, auf Basis der Messsignale des Messschaltkreises (6) ein Diagramm oder eine schematische Darstellung einer Knochenstruktur anzuzeigen, bei dem / der Werten der Eindringtiefe gemittelte Werte mehrerer bestimmter Motorstromwerte, zu den Motorstromwerten korrelierender oder im Wesentlichen proportionaler Drehmomentwerte oder in Knochenqualitätsklassen zusammengefasste Qualitäten eines Knochens zugeordnet sind.

12. Medizinische, insbesondere dentale, Vorrichtung (1) nach einem der vorstehenden Ansprüche, **dadurch gekennzeichnet, dass**
die Vorrichtung (1) des Weiteren einen motorischen Antrieb (5) und ein mit dem Gewindeelement (3) verbindbares oder verbundenes Handgriffelement (9) aufweist, das mit dem motorischen Antrieb (5) kuppelbar ist oder den motorischen Antrieb (5) umfasst.

13. Medizinische, insbesondere dentale, Vorrichtung (1) nach einem der vorstehenden Ansprüche, **dadurch gekennzeichnet, dass**
die Steuervorrichtung (2) eine Auswerteschaltung (10) aufweist, die ausgebildet ist, die von dem Messschaltkreis (6) bestimmten Motorstromwerte oder die zu den Motorstromwerten korrelierenden oder im Wesentlichen proportionalen Drehmomentwerte mit Vergleichswerten zur Bestimmung der Qualität eines Knochens zu vergleichen und / oder zu Knochenqualitäts-Klassen zusammenzufassen und / oder Knochenqualitäts-Klassen zuzuordnen.

14. Medizinische, insbesondere dentale, Vorrichtung (1) nach einem der vorstehenden Ansprüche, **dadurch gekennzeichnet, dass**
die Steuervorrichtung (2) des Weiteren ausgebildet ist, die von dem motorischen Antrieb (5) benötigte oder aufgewendete Energie, Arbeit oder Leistung während des Schneidens des Gewindes in einen Knochen zu ermitteln und ein entsprechendes Anzeigesignal über die zweite Kontaktvorrichtung (2B) an die Anzeigeeinheit (4) zu übertragen, so dass die Anzeigeeinheit (4) auf Basis des übertragenen Anzeigesignals die während des Schneidens des Gewindes benötigte oder aufgewendete Energie, Arbeit oder Leistung wiedergibt.

15. Medizinische, insbesondere dentale, Vorrichtung (1) nach einem der vorstehenden Ansprüche, **dadurch gekennzeichnet, dass**
das rotierende Gewindeelement (3) einen Gewindeschneider oder ein Implantat umfasst.

## Claims

1. A medical, in particular dental, device (1) for determining the quality of a bone by cutting a thread into the bone by a rotating threaded element (3) having at least one cutting edge, wherein the medical, in particular dental, device (1) comprises a control unit (2) which comprises a first electrical contact device (2A) for connection to a motor drive (5), a second contact device (2B) for connection to a display unit (4) and a measurement circuit (6), which is operatively connected to the first electrical contact device (2A) and to the second contact device (2B), wherein the measurement circuit (6) is configured:

(i) to determine values of the motor current via the first electrical contact device (2A), the motor current being supplied to the motor drive (5) to rotatably drive the threaded element (3) that can be connected to the motor drive (5), wherein the motor current values correlate with or are substantially proportional to transmitted torque values of the motor drive (5), and wherein the motor current values or torque values are a measure of the quality of a bone,
(ii) to monitor and/or determine the depth of penetration of the threaded element (3) into the bone, and
(iii) to generate measurement signals which show the relationship between the determined motor current values, the torque values which correlate with or are substantially proportional to the motor current values or the quality of a bone derived from the determined motor current values or the torque values and the depth of penetration, and to transmit these measurement signals via the second contact device (2B) to the display unit (4), so that the display unit (4) displays on the basis of the transmitted measurement signals the relationship between the depth of penetration and at least one of the following parameters, namely the determined motor current values, the torque values which correlate with or are substantially proportional to the motor current values or the quality of a bone,

**characterized in that**
the control unit (2) is configured to begin the monitoring, determination or a recording of the depth of penetration or of the progressive penetration of the threaded element (3) into the bone only after reaching or exceeding a predetermined motor current threshold or torque threshold.

**2.** The medical, in particular dental, device (1) according to claim 1, **characterized in that** the measurement circuit (6) is configured to process angle of rotation values of a rotor (5) of the motor drive (5) in order to monitor and/or determine the depth of penetration of the threaded element (3) into the bone.

**3.** The medical, in particular dental, device (1) according to claim 1 or 2, **characterized in that**
the measurement circuit (6) is configured to take into account the transmission ratio, in particular including the degree of efficiency, of a gear unit (7) of the medical, in particular dental, device (1) arranged between the motor drive (5) and the threaded element (3) in order to monitor and/or determine the depth of penetration of the threaded element (3) into the bone in order.

**4.** The medical, in particular dental, device (1) according to any one of the preceding claims, **characterized in that**
the measurement circuit (6) is configured to take into account at least one property of the threaded element (3), for example, the slope of the at least one cutting edge, the shape of the cutting edge or the outer shape of the threaded element (3), in order to monitor and/or determine the depth of penetration of the threaded element (3) into the bone in order.

**5.** The medical, in particular dental, device (1) according to any one of the preceding claims, **characterized in that**
at least one predetermined depth of penetration is stored in the control unit (2).

**6.** The medical, in particular dental, device (1) according to any one of the preceding claims, **characterized in that**
a plurality of predetermined depths of penetration are stored in the control unit (2) which can be selected by the user through an actuating element (8).

**7.** The medical, in particular dental, device (1) according to claim 5 or 6, **characterized in that**
the control unit (2) is configured to compare the monitored and/or determined depth of penetration of the threaded element (3) with the at least one predetermined depth of penetration and, on reaching or exceeding the at least one predetermined depth of penetration, to stop the motor drive (5) and/or to change its direction of rotation.

**8.** The medical, in particular dental, device (1) according to any one of the preceding claims, **characterized in that**
the control unit (2) is configured to repeatedly compare the instantaneous motor current value determined by the measurement circuit (6) or torque value with the predetermined motor current threshold or torque threshold until reaching the predetermined motor current threshold or torque threshold.

**9.** The medical, in particular dental, device (1) according to any one of the preceding claims, **characterized by**
a display unit (4) that is communicatively connected to the second contact device (2B) and which displays the relationship between the depth of penetration and at least one of the following parameters, namely the determined motor current values, the torque values which correlate with or are substantially proportional to the motor current values or the quality of a bone.

**10.** The medical, in particular dental, device (1) according to claim 9, **characterized in that** the display unit (4) displays graphically the relationship between the depth of penetration and at least one of the following parameters, namely the determined motor current values, the torque values which correlate with or are substantially proportional to the motor current values or the quality of a bone, for example, in the form of a diagram or a schematic representation of a bone structure.

**11.** The medical, in particular dental, device (1) according to claim 10, **characterized in that**
the display unit (4) is configured to display a diagram or a schematic representation of a bone structure on the basis of the measurement signals of the measurement circuit (6) such that to each value of the depth of penetration a value of the determined motor current, a torque value that correlates with or is substantially proportional therewith or a quality of the bone is assigned, or the display unit (4) is configured to display a diagram or a schematic representation of a bone structure on the basis of the measurement signals of the measurement circuit (6) such that to values of the depth of penetration averaged values of a plurality of determined motor current values, torque values correlating with or substantially proportional to the motor current values or qualities of a bone combined into bone quality classes are assigned.

**12.** The medical, in particular dental, device (1) according to any one of the preceding claims, **characterized in that**
the device (1) additionally comprises a motor drive (5) and a handle element (9) that is or can be connected to the threaded element (3) and which is connectable to the motor drive (5) or comprises the motor drive (5).

**13.** The medical, in particular dental, device (1) according to any one of the preceding claims, **characterized in that**
the control unit (2) comprises an evaluation circuit (10) which is configured to compare the motor current values determined by the measurement circuit (6) or the torque values which correlate with or are substantially proportional to the motor current values with comparative values for determining the quality of a bone and/or to combine them into bone quality classes and/or to assign them to bone quality classes.

**14.** The medical, in particular dental, device (1) according to any one of the preceding claims, **characterized in that**
the control unit (2) is additionally configured to determine the energy, work or power required or expended by the motor drive (5) during the cutting of the thread in a bone and to transmit a corresponding display signal via the second contact device (2B) to the display unit (4), so that the display unit (4) displays the energy, work or power required or expended during the cutting of the thread on the basis of the transmitted display signal.

**15.** The medical, in particular dental, device (1) according to any one of the preceding claims, **characterized in that**
the rotating threaded element (3) comprises a thread cutter or an implant.

## Revendications

**1.** Dispositif (1) médical, en particulier dentaire, pour la détermination de la qualité d'un os par taillage d'un filetage dans l'os au moyen d'un élément fileté rotatif (3) avec au moins une arête vive, dans lequel le dispositif (1) médical, en particulier dentaire, comprend un dispositif de commande (2) qui présente un premier dispositif de contact électrique (2A) pour le raccordement à un entraînement motorisé (5), un deuxième dispositif de contact (2B) pour le raccordement à une unité d'affichage (4) et un circuit de mesure (6), lequel est relié de manière fonctionnelle au premier dispositif de contact électrique (2A) et au deuxième dispositif de contact (2B), dans lequel le circuit de mesure (6) est réalisé pour :

(i) via le premier dispositif de contact électrique (2A), déterminer des valeurs du courant de moteur avec lequel l'entraînement motorisé (5) est alimenté pour l'entraînement en rotation de l'élément fileté (3) pouvant être relié à l'entraînement motorisé (5), dans lequel les valeurs de courant de moteur sont en corrélation avec, ou sensiblement proportionnelles à des valeurs de couple transmises de l'entraînement motorisé (5) et dans lequel les valeurs de courant de moteur ou valeurs de couple sont une mesure pour la qualité d'un os,
(ii) surveiller et/ou déterminer la profondeur de pénétration de l'élément fileté (3) dans l'os et
(iii) générer des signaux de mesure, lesquels mettent en rapport les valeurs de courant de moteur déterminées, les valeurs de couple en corrélation avec les valeurs de courant de moteur ou sensiblement proportionnelles à celles-ci, ou la qualité d'un os déduite des valeurs de courant de moteur déterminées ou des valeurs de couple, avec la profondeur de pénétration, et transmettre ces signaux de mesure via le deuxième dispositif de contact (2B) à l'unité d'affichage (4) de sorte que, sur la base des signaux de mesure transmis, l'unité d'affichage (4) restitue le rapport entre la profondeur de pénétration et au moins l'un des paramètres suivants, à savoir les valeurs de courant de moteur déterminées, les valeurs de couple en corrélation avec les valeurs de courant de moteur ou sensiblement proportionnelles à celles-ci, ou la qualité d'un os,

**caractérisé en ce que**
le dispositif de commande (2) est réalisé pour commencer avec la surveillance, la détermination, ou avec un enregistrement de la profondeur de pénétration ou de la progression de la pénétration de l'élément fileté (3) dans l'os seulement après avoir atteint ou dépassé une valeur de seuil de courant de moteur ou valeur de seuil de couple prédéterminée.

**2.** Dispositif (1) médical, en particulier dentaire selon la revendication 1, **caractérisé en ce que**
le circuit de mesure (6) est réalisé, pour la surveillance et/ou détermination de la profondeur de pénétration de l'élément fileté (3) dans l'os, pour traiter des valeurs d'angle de rotation d'un rotor (5A) de l'entraînement motorisé (5).

**3.** Dispositif (1) médical, en particulier dentaire selon la revendication 1 ou 2, **caractérisé en ce que**
le circuit de mesure (6) est réalisé, pour la surveillance et/ou détermination de la profondeur de pénétration de l'élément fileté (3) dans l'os, pour prendre en compte le rapport de transmission, y compris en particulier l'efficacité, d'une unité d'engrenage (7) du dispositif (1) médical, en particulier dentaire, disposée entre l'entraînement motorisé (5) et l'élément fileté (3).

**4.** Dispositif (1) médical, en particulier dentaire selon l'une des revendications précédentes, **caractérisé en ce que**
le circuit de mesure est réalisé, pour la surveillance et/ou détermination de la profondeur de pénétration de l'élément fileté (3) dans l'os, pour prendre en compte au moins une propriété de l'élément fileté (3), par exemple le pas de l'au moins une arête vive, la forme de l'arête vive ou la forme extérieure de l'élément fileté (3).

**5.** Dispositif (1) médical, en particulier dentaire selon l'une des revendications précédentes, **caractérisé en ce que**,
dans le dispositif de commande (2), au moins une profondeur de pénétration prédéterminée est enregistrée.

**6.** Dispositif (1) médical, en particulier dentaire selon l'une des revendications précédentes, **caractérisé en ce que**
dans le dispositif de commande (2), plusieurs profondeurs de pénétration prédéterminées sont enregistrées, lesquelles peuvent être sélectionnées par l'utilisateur via un élément de réglage (8).

**7.** Dispositif (1) médical, en particulier dentaire selon la revendication 5 ou 6, **caractérisé en ce que**
le dispositif de commande (2) est réalisé pour comparer la profondeur de pénétration surveillée et/ou déterminée de l'élément fileté (3) avec l'au moins une profondeur de pénétration prédéterminée et, en cas d'atteinte ou de dépassement de l'au moins une profondeur de pénétration prédéterminée, pour arrêter l'entraînement motorisé (5) et/ou modifier son sens de rotation.

**8.** Dispositif (1) médical, en particulier dentaire selon l'une des revendications précédentes, **caractérisé en ce que**
le dispositif de commande (2) est réalisé pour, jusqu'à l'atteinte de la valeur de seuil de courant de moteur ou valeur de seuil de couple prédéterminée, comparer de manière répétée la valeur de courant de moteur ou valeur de couple actuelle déterminée par le circuit de mesure (6) avec la valeur de seuil de courant de moteur ou valeur de seuil de couple prédéterminée.

**9.** Dispositif (1) médical, en particulier dentaire selon l'une des revendications précédentes, **caractérisé par**
une unité d'affichage (4) raccordée de façon communicante au deuxième dispositif de contact (2B), laquelle restitue le rapport entre la profondeur de pénétration et au moins l'un des paramètres suivants, à savoir les valeurs de courant de moteur déterminées, les valeurs de couple en corrélation avec les valeurs de courant de moteur ou sensiblement proportionnelles à celles-ci, ou la qualité d'un os.

**10.** Dispositif (1) médical, en particulier dentaire selon la revendication 9, **caractérisé en ce que**
l'unité d'affichage (4) représente graphiquement le rapport entre la profondeur de pénétration et au moins l'un des paramètres suivants, à savoir les valeurs de courant de moteur déterminées, les valeurs de couple en corrélation avec les valeurs de courant de moteur ou sensiblement proportionnelles à celles-ci, ou la qualité d'un os, par exemple sous la forme d'un diagramme ou d'une représentation schématique d'une structure osseuse.

**11.** Dispositif (1) médical, en particulier dentaire selon la revendication 10, **caractérisé en ce que**
l'unité d'affichage (4) est réalisée, sur la base des signaux de mesure du circuit de mesure (6), pour afficher un diagramme ou une représentation schématique d'une structure osseuse dans lequel / laquelle on associe, à chaque valeur de la profondeur de pénétration, une valeur du courant de moteur déterminée, une valeur de couple en corrélation avec celle-ci ou sensiblement proportionnelle à celle-ci, ou une qualité de l'os, ou que l'unité d'affichage (4) est réalisée, sur la base des signaux de mesure du circuit de mesure (6), pour afficher un diagramme ou une représentation schématique d'une structure osseuse dans lequel / laquelle on associe, à des valeurs de la profondeur de pénétration, des valeurs moyennes de plusieurs valeurs de courant de moteur déterminées, des valeurs de couple en corrélation avec les valeurs de courant de moteur ou sensiblement proportionnelles à celles-ci, ou des qualités d'un os regroupées dans des classes de qualité d'os.

**12.** Dispositif (1) médical, en particulier dentaire selon l'une des revendications précédentes, **caractérisé en ce que**
le dispositif (1) présente en outre un entraînement motorisé (5) et un élément de poignée (9) relié, ou pouvant être relié à l'élément fileté (3), lequel peut être couplé avec l'entraînement motorisé (5) ou bien englobe l'entraînement motorisé (5).

**13.** Dispositif (1) médical, en particulier dentaire selon l'une des revendications précédentes, **caractérisé en ce que**
le dispositif de commande (2) présente un circuit d'évaluation (10) qui est réalisé pour comparer les valeurs de courant de moteur déterminées par le circuit de mesure (6) ou les valeurs de couple en corrélation avec les valeurs de courant de moteur ou sensiblement proportionnelles à celles-ci, avec des valeurs de comparaison pour la détermination de la qualité d'un os et / ou pour les regrouper en classes de qualité d'os et/ou les affecter à des classes de qualité d'os.

**14.** Dispositif (1) médical, en particulier dentaire selon l'une des revendications précédentes, **caractérisé en ce que**
le dispositif de commande (2) est en outre réalisé pour déterminer l'énergie nécessaire pour l'entraînement motorisé (5), ou utilisée par celui-ci, le travail ou la puissance pendant le taillage du filetage dans un os, et transmettre un signal d'affichage correspondant via le deuxième dispositif de contact (2B) à l'attention de l'unité d'affichage (4) de sorte que sur la base du signal d'affichage transmis, l'unité d'affichage (4) restitue l'énergie, le travail ou la puissance nécessaire ou utilisé(e) pendant le taillage du filetage.

**15.** Dispositif (1) médical, en particulier dentaire selon l'une des revendications précédentes, **caractérisé en ce que**
l'élément fileté rotatif (3) comprend un outil à fileter ou un implant.

FIG. 1

1
11
2B
4
9A
9
2
9B
13
3
12
7
5A
5
14
2A
8
10
6

FIG. 2

Drehmoment [Nmm]
Tiefe [mm]

FIG. 3

Q1
Q2
Q3
Q4
Knochenqualität
0
1
2
3
4
5
6
7
8
Tiefe [mm]

FIG. 4

Tiefe
16
Q1
Q2
Q3
15

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2014076653 A1
- US 20110245833 A1
- WO 2013050851 A1